# EUROPEAN PATENT SPECIFICATION

(11) **EP 3 975 881 B1**
(45) Date of publication and mention of the grant of the patent: **02.04.2025**
(21) Application number: 20730005.4
(22) Date of filing: 28.05.2020
(51) Int. Cl.: A61B 17/17, A61B 17/15, A61B 17/86

(54) **OSTEOTOMY DEVICE**
VORRICHTUNG ZUR OSTEOTOMIE
DISPOSITIF D'OSTÉOTOMIE

(30) Priority: 31.05.2019 GB 201907781
(43) Date of publication of application: 06.04.2022
(73) Proprietor: Innovation Advances Limited, Hemel Hempstead, Hertfordshire HP3 9LB (GB)
(72) Inventor: SAKSIDA, Kristian, Northwood, Christchurch 8051 (NZ)
(74) Representative: Page White Farrer
(86) International application number: PCT/EP2020/064925
(87) International publication number: WO 2020/239959

(56) References cited:
- FR-A1- 2 676 353
- US-A- 4 750 481
- US-A- 5 540 695
- US-A1- 2016 089 166

## Description

### Field of the invention

The present disclosure relates to a surgical device suitable for use in methods for performing bone osteotomies.

### Background

An osteotomy surgery involves the cutting of a bone in order to change its length, shape or alignment within the body. In particular, osteotomies can be used to realign the axis of the bone and move the weight bearing axis that passes through the end of a bone. Such surgeries can be helpful to address problems such as joint wear (including the loss of cartilage) in a particular area of a joint surface, and to relieve the associated pain and arthritis. These surgeries aim to increase the life of a joint and therefore delay or prevent the need for a partial or total joint replacement operation.

The osteotomy surgery can involve making a cut to the bone and then opening the cut to create an open wedge shape or aperture. This technique is known as "an opening wedge osteotomy". An osteotomy surgery can also involve making at least two cuts to the bone to create a wedge of bone which is then removed. The cut surfaces of the bone are then brought together. This is known as "a closing wedge osteotomy".

Common examples of osteotomies in knee surgery are a high tibial osteotomy (HTO) and distal femoral osteotomy (DFO) surgeries. In particular, the knee is divided into 3 compartments - the anterior or knee cap compartment, the medial or inner compartment, and the outer or lateral compartment. The most common procedure, an HTO, is used most typically when a patient presents with a varus or bowed alignment at the knee with damage primarily in the inner or medial compartment. Here the bone is cut in the upper part and an opening wedge created which pushes the weight bearing line from the medial side of the knee to the lateral or outer compartment. Less typically a single cut is performed from the outer side or lateral side of the upper tibia which would push the weight bearing line into the medial or inner compartment of the knee.

It is also possible to carry out a "closing wedge" in the upper or proximal tibia from either the medial or lateral side of the bone. Here the osteotomy is performed to change the angulation and alignment at the knee by making two cuts from either the inner or medial side of the bone which would bring the leg towards a varus alignment. Conversely a closing wedge can be carried out on the outer side of the bone, and this draws the alignment towards the lateral side and would move the weight bearing line travelling through the knee into the outer compartment of the knee or lateral side.

The converse is where a patient presents with damage on the outer or lateral side of the knee and valgus alignment or a knock-kneed appearance. So in summary in closing wedge surgery a wedge of bone is removed from the tibia or femur and the cut edges fixed together (usually with a metal plate or pins), or in an opening wedge osteotomy, the bone surfaces either side of a cut in the tibia or femur are forced apart to create a wedge-shaped aperture. The bone is held by an external plate that is applied to the bone. The surgery may be carried out with the addition of material into the gap, either bone graft or artificial material, and then adding an external plate to the outer side of the bone to bridge above and below the osteotomy. These changes in the tibia and femur allow surgeons to move the weight bearing line or axis though the knee away from the damaged area and load the knee away from the damage through the normal compartment of the knee and thereby change the alignment of the joint.

The realignments that are performed during these knee surgeries, and others like them performed on different bones/joints around the body, rely on the formation of cuts in the bone to establish the opening or closing wedges. These cuts do not extend across the full width of the bone. Instead an area of uncut bone is retained on the opposite side of the bone to the surface where the bone cut is started. This area of uncut bone is termed the "bone hinge" as it is this area of bone that will be stretched or squeezed as the bone wedge is removed (during a closing wedge osteotomy) or the wedge aperture created (during an opening wedge osteotomy). The osteotomy bone hinge is known to play an important role in stabilizing the bone arrangement after the osteotomy operation. In particular, it is known that tiny fractures are created in the bone hinge that extend towards the opposite cortex from where the osteotomy cut starts. To enhance stability care is taken during surgery to ensure that the bone hinge is retained and is as intact as possible.

As life expectancy increases, patients who have had an initial joint replacement are increasingly facing the possibility that they may at some stage in later life require a repeat of the joint replacement. The success rates for such repeat replacements are lower than those for the initial replacement. Therefore, surgeries such as osteotomies that delay or prevent the need for initial joint replacement are of increasing interest.

US 4 750 481 A discloses osteotomy devices and methods for correcting bone deformities, FR 2 676 353 A1 discloses plates for tibial osteotomies.

### Summary

The present invention provides a device and a surgical package as defined in the appended claims. The methods disclosed herein do not form part of the invention.

In particular, the present invention provides a device suitable for use during surgery to direct the insertion of a guide or an implant across a bone hinge, wherein the bone hinge is adjacent to a cut made in a bone during the surgery, the device comprising:
(i) a main body comprising or consisting of a first arm, wherein the first arm comprises a first opening for receiving the guide or the implant; and
(iii) an insertion part having a first end and a second end, the first end comprising an elongated portion which is suitable for insertion into the cut, and the second end comprising an attachment part for reversibly attaching the insertion part to the main body;
wherein when the elongated portion is inserted into the cut and the insertion part is attached to the main body, the first opening is configured to direct the insertion of the guide or the implant into the bone from a first point on the outer surface across the bone hinge.

In particular, the device is for use in a surgical method for compressing a bone hinge created during surgery to re-align a bone in a subject.

In accordance with the presence disclosure, the method comprises:
inserting an implant comprising a first end and an opposing second end into the bone from a first point on an outer surface of the bone, wherein the first end remains at the first point and the second end is inserted across the bone hinge to the bone on the opposite side of the bone hinge; and
applying a compressive force between the first end and the second end across the bone hinge.

In the method above, an implant, which traverses through the bone hinge and which is to remain in the bone after the surgery, is used to apply a compressive force across the bone hinge. The compression is directly applied across the bone hinge by the implant in order to stabilize the hinge and promote stability of the osteotomy and thereby accelerate healing, thus improving the outcome of the surgery. This is advantageous over the methods of the prior art that utilise devices (e.g. plates and fixings) attached to the bone on the opposite side of the bone to the bone hinge and therefore apply only indirect compression.

The device of the invention is suitable for assisting in the insertion of the implant (or a guide which can be used to insert the implant) at the correct position.

Still further the present disclosure provides a sterile surgical package comprising the main body and the insertion part of the device described above, optionally wherein the package comprises one or more of: (i) an implant; and (ii) at least one guide,
wherein the implant is suitable for suitable for applying compression across a bone hinge, and wherein the guide is a wire or pin..

Preferred features of these aspects of the disclosure are defined in the dependent claims.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Nor is the claimed subject matter limited to implementations that solve any or all of the disadvantages noted herein.

### Description of the Drawings

To assist understanding of the present disclosure and to show how embodiments may be put into effect, reference is made, by way of example only, to the accompanying drawings in which:
Figure 1A is a diagram showing the position of the implant in an example of the present disclosure. The area within the dashed box is shown in Figure 1B. The Figure shows: (1) bone hinge; (2) implant; (3) first end of implant; (4) opposing second end of implant; (5) first point on outer surface of bone; (6) cut; (7) second point on outer surface of bone; (8) aperture; and (9) tibia.
Figure 1B is a diagram showing an expanded version of the boxed area in Figure 1A. The Figure shows: (1) bone hinge; (2) implant; (6) cut; (20) direction of implant; (21) direction of cutting at termination of cut; and (30) theoretical line representing shortest distance between point at which the cut terminates and the bone cortex.
Figure 2 is a diagram showing how the device of the present disclosure may be utilised to position a K-wire (which is to be used as a guide) across a bone hinge during an osteotomy. The Figure shows: (1) bone hinge; (10) guide; (11) first arm; (12) first opening; (13) insertion part; (14) elongate portion; (15) second arm; (16) second opening; and (17) joining part.
Figure 3A is a side view of the main body comprising first and second arms in an example of the device of the present disclosure. The Figure shows: (19) main body; (11) first arm; and (15) second arm.
Figure 3B is an upper lateral view of the main body comprising first and second arms in an example of the device of the present disclosure. The Figure shows: (19) main body; (11) first arm; (15) second arm; and (16) second opening.
Figure 3C is a view of the insertion part in an example of the device of the present disclosure. The Figure shows: (13) insertion part; (14) elongate portion; and (18) slit.
Figure 4A is a diagram showing a bone wedge formed in a femur during a closing wedge osteotomy surgery. The Figure shows: (1) bone hinge; (7) second point on outer surface of bone; (23) distal femur; (24) cartilage; (25) bone wedge; and (26) centre of osteotomy.
Figure 4B is a diagram showing the femur of Figure 4A once the bone wedge is removed and the cut edges of the bone are brought together. The Figure shows: dashed lines marked as (27) are previous position of distal femur, while the horizontal arrows mark the change in position of the distal femur; and (28) cut surfaces of bone brought together after bone wedge shown in Figure 4A is removed.
Figure 4C is a diagram showing a cross section of the femur of Figure 4B with the implant inserted. The Figure shows: (2) implant; (5) first point on outer surface of bone; dashed lines marked as (27) are previous position of distal femur, while the horizontal arrows mark the change in position of the distal femur; (28) cut surfaces of bone brought together; and (29) screw threads.
Figure 5 is a diagram showing the position of an implant in 3D in a femur with an opening wedge osteotomy; The Figure shows: (2) implant; (8) aperture; and (23) distal femur.

### Detailed Description

Various embodiments are described in detail and may be further illustrated by the provided Figures. As used in the description herein and throughout the claims that follow, the meaning of "a," "an," and "the" includes plural reference unless the context clearly dictates otherwise.

The terms used in this specification generally have their ordinary meanings in the art, within the context of the present disclosure, and in the specific context where each term is used. Certain terms that are used are discussed below, or elsewhere in the specification, to provide additional guidance to the practitioner in describing the present methods and devices.
within one aspect, the present description provides a method for performing osteotomy surgery to re-align a bone in a subject.

Specifically, the method is for compressing a bone hinge created during surgery to re-align a bone in a subject, the method comprising:
inserting an implant comprising a first end and an opposing second end into the bone from a first point on an outer surface of the bone, wherein the first end remains at the first point and the second end is inserted across the bone hinge to the bone on the opposite side of the bone hinge; and
applying a compressive force between the first end and the second end across the bone hinge.

The method can be used in any osteotomy surgery involving the creation of a bone hinge. In particular, these may be any opening wedge or closing wedge osteotomies. The bone may be a bone in a part of an upper limb, including a bone in the shoulder joint, elbow, or wrist, or a small bone of the hand, or may be a bone in a lower limb, including a bone in the hip, knee and ankle, as well as a small bone of the foot. Preferably the bone is a long bone, more preferably the bone is the tibia or the femur. More preferably the osteotomy is a high tibial or distal femoral osteotomy, including both medial and lateral approaches and both closing and opening wedge procedures. Most preferably the osteotomy is a medial opening wedge high tibial osteotomy.

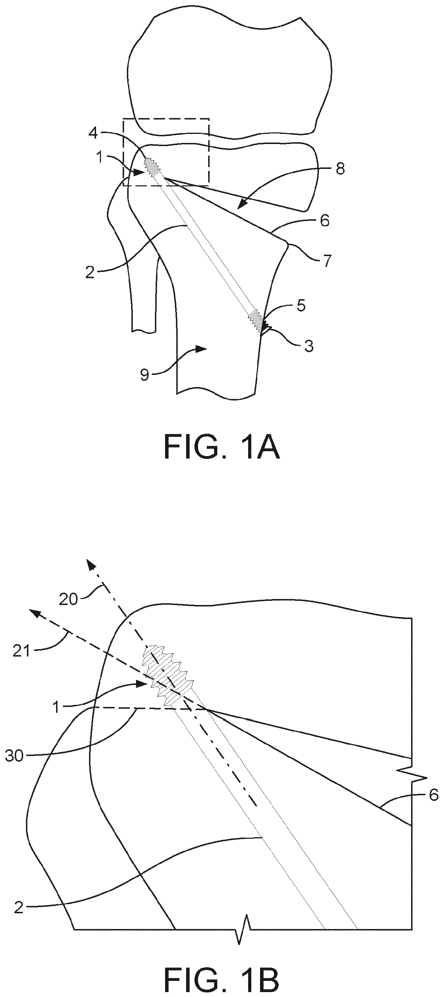

The implant is inserted into the bone relative to a cut made in the bone from a second point on the outer surface, which cut has resulted in the creation of the bone hinge. The method of the disclosure may comprise making this cut. The cut may be straight or curved (such as in a crescent-type osteotomy). Preferably the cut is made in a straight line (i.e. along a cutting plane). The cut normally extends from a second point on the outer surface towards the opposite side of the bone. In a preferred example the cut is made obliquely towards a joint. In particular, this cut ends under the joint at the bone hinge, which is discussed further below. Figures 1A and 1B show an example of the position of the implant (2) inserted in a tibia (9) across a bone hinge (1) relative to the cut (6). In particular, the cut is made from a second point (7) on the outer surface of a tibia (9). Where the osteotomy is an open wedge high tibial osteotomy this cut is made from the medial surface of the tibia.

The cut in the bone from the second point on the outer surface creates a first segment of bone and a second segment of bone that are separated by the cut, i.e. the cut separates the bone into a first segment on one side of the cut, and a second segment on the other side of the cut.

The cut is a partial (or incomplete) cut. It does not result in the first segment and the second segment becoming separate pieces i.e. it does not extend across the entire width of the bone. In particular, the cut terminates close to the cortex at a termination point (or hinge point). This would normally be towards the opposite side of the bone to the second point from which the cut was started. It is possible to have a termination or hinge point anywhere along the line of the cut but the closer the termination or hinge point is to the cortex the easier it is to open or close the osteotomy and change the angulation of the bone.

The distance of the termination point from the cortex on the opposite side will depend on the size of the bone. In the normal case where the cut extends towards the opposite side of the bone to the second point from which the cut was started, the cut usually extends across 70 to 95%, preferably 80 to 95%, more preferably 85 to 95% of the width of the bone at the point at which the cut terminates leaving 5 to 30%, preferably 5 to 20%, more preferably 5 to 15% of the bone uncut. For a tibial osteotomy the cutting can be stopped at a point approximately 5-10 mm ± 1mm, preferably 5 to 8 mm± 1mm from the bone cortex at the opposite side.

Since the bone is not completely cut there is an area of uncut bone between the point at which the cut terminates and the cortex, which joins the first and second segments of bone. This area of uncut bone is the bone hinge and lies along a theoretical line (or plane) that represents the shortest distance between the point at which the cut terminates and the outer surface of the bone (the cortex). (This theoretical line is marked on Figure 1B as dashed line 30). In one example, the bone hinge can be defined as a section (or slice) of bone that extends at least 1 mm, at least 2 mm, or at least 3 mm either side of this theoretical line.

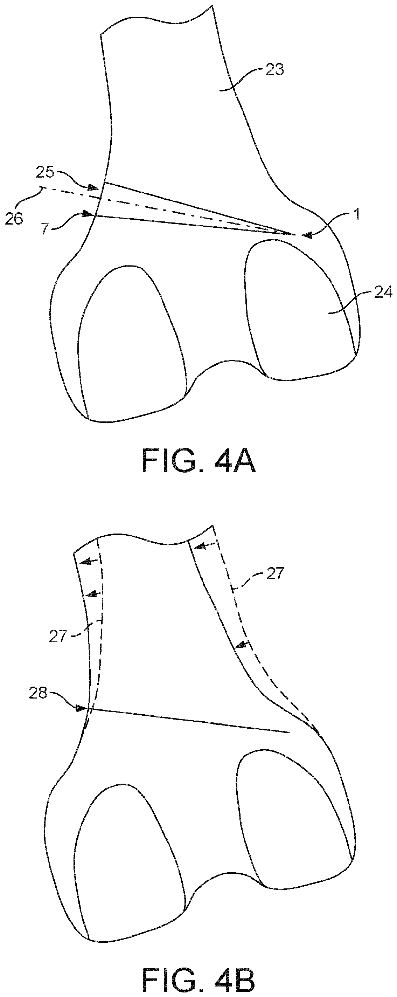

As a result of the bone hinge of uncut bone the first and second segments of bone either side of the cut remain joined together. The bone hinge (1) and cut (6) are shown in Figures 1A and 1B, along with the direction of cutting at the termination of the cut (dashed arrow 21). The bone hinge is the area of bone that will become stretched or squeezed during the opening or closing of the wedge, which is discussed further below. Therefore, the length of the bone hinge between the point at which the cut terminates and the cortex must be short enough to allow some mobility of the first and second segments relative to each other, i.e. short enough to allow the first and second segments of bone to be moved apart to create an aperture (as in an opening wedge osteotomy - an example of which is shown in Figures 1A and 1B) or to allow a wedge of bone to be removed and the cut surfaces of bone brought together (as in a closing wedge osteotomy - an example of which is shown in Figures 4A to 4C). As noted above, the length of the bone hinge depends on the size of the bone and in a tibial osteotomy can be 5-10 mm in length (measuring from the termination point of the cut to the nearest point on the outer surface of the bone).

Any bone saw may be used to make the osteotomy cuts but most commonly an oscillating saw blade or reciprocating saw can be used to make the cut. Other suitable cutting devices can be used such as chisels or burrs. It is known in the art to use general cutting guides or patient specific cutting guides (based on initial X-ray or fluoroscope imaging) to ensure that cutting is accurate and the cut does not extend across the entire width of the bone. Guide wires, such as Kirshner (K-wires) wires, can be used to guide the cut. In a tibial osteotomy before cutting the bone, the surgeon places a guide wire or more commonly 2 guide wires under X-ray control or by using patient specific guiding blocks that allow the wires to be placed in the ideal location as previously determined by pre-operative planning. The guide wire is placed at the appropriate starting point on the inner or medial cortex of the bone in an upper medial high tibial osteotomy. In an upper lateral high tibial osteotomy the wire or wires are initially placed on the outer or lateral side of the bone. The guide wires are then directed in an upward direction towards the other side of the bone - in a medial high tibial osteotomy towards the lateral side of the bone or in a lateral HTO towards the medial side of the bone. The cutting tool is then used to cut the bone as far as the bone hinge point which, as indicated above, is approximately 5-10 mm away from the opposite cortex or outer part of the bone.

The method of the disclosure may comprise re-aligning the bone by: (i) separating the first segment of bone form the second segment of bone to create an aperture (e.g. part (8) in the example shown in Figure 1A) and fixing the position of the first and second segments of bone; or (ii) forming an aperture by cutting and removing a section of bone from the first or second segment of bone on a side of the first cut, closing the aperture and fixing the aperture in a closed position.

In particular, where the method of surgery is an opening wedge osteotomy (i.e. method (i) above), the method comprises separating the first segment of bone from the second segment of bone to create an aperture. Techniques for separating are known in the art. In particular, wedge-shaped pieces can be tapped one by one into the cut in order to gradually widen the aperture. Once the aperture is of the correct size, this arrangement is then fixed. This can be done by internal or external fixing devices, such as plates and pins. The aperture may be filled with bone graft or artificial material.

When the method of surgery is a closing wedge osteotomy (i.e. method (ii) above) the method comprises making a second cut such that the first cut and the second cut meet at the termination point of the first cut. This allows a wedge of bone to be removed and the cut surfaces of the bone brought together and held in place by external fixing devices, such as plates and pins.

According to the present disclosure the method comprises inserting an implant comprising a first end and an opposing second end into the bone from a first point on the outer surface of the bone, wherein the first end remains at the first point and the second end is inserted across the bone hinge to the bone on the opposite side of the bone hinge. An example in shown in Figure 1A, where the implant (2) is shown with a first end (3) and an opposing second end (4) extending from a first point (5) on the outer surface of the bone across the bone hinge (1)).

In particular, the implant extends from the first point on the outer surface of the bone at a position separate from the second point (from which the partial bone cut is made) and along a line (direction (20) in the example shown in Figure 1B) that crosses the bone hinge. In particular, it intersects with a plane that represents the direction of cutting at the termination point of the cut (shown by direction (21) in the example shown in Figure 1B) and also which intersects with the theoretical line (or plane) that represents the shortest distance between the point at which the cut terminates and the cortex (line (30) in the example shown in Figure 1B). As such, the implant extends across the bone hinge. In particular, the implant is inserted across the bone hinge obliquely or at 90 °, in order to effect compression. The implant can be placed from the same side of the bone as the hinge from above or below or be placed from the opposite side of the bone from the hinge, again from below or above. The positioning of the implant can be selected in order to achieve the largest angle possible across the bone hinge, in order to achieve as much compression across the bone hinge as possible given the anatomical constraints set by the specific osteotomy being performed. In particular, the implant may be inserted at an angle of at least 15° to the bone hinge, preferably at an angle of at least 20°. The angle may be 20° to 90°, or 20° to less than 90°. The angle may be 25° to 90°, 25° to less than 90°, 25° to 80°, 25° to 70° or 25° to 60°. The angle may be 30° to 90°, 30° to 80°, 30° to 70° or 30° to 60°. For a high tibial osteotomy in which the implant is inserted from the same side of the bone as the cut an angle of 25° to 35°, 28° to 32° or approximately 30° can be used. In particular, these angles may be taken as the angle between the implant and the theoretical line (or plane) that represents the shortest distance between the point at which the cut terminates and the cortex.

The implant has a first end, which is retained at the first point on the surface of the bone, and an opposing second end that is inserted across the bone hinge and into the area of bone on the opposite side of the bone hinge to the first point. In a preferred example the implant extends at least 1-2 mm, at least 5 mm, or between 10-15 mm beyond the theoretical line (or plane) that represents the shortest distance between the point at which the cut terminates and the cortex. However, the extent to which the second end extends beyond this is not strictly limited. As discussed below, the second end may extend to the exterior of the bone (except where this would result in the second end perforating the joint surface). Where the second end extends to the exterior of the bone the second end should not be significantly palpable through the skin. However, preferably the second end remains inside the bone and does not exit the bone.

If the method uses a guide wire to assist in inserting the implant, as discussed further below, the degree of insertion can be measured under fluoroscopic control with another K-wire of the same length and then the overlap of the wires (one in the bone, one at the cortex) can be measured.

The implant can be any part suitable for applying compression across the bone hinge. The implant may be a screw. Preferably the screw is a compression screw. Such a compression screw may have a stepped thread pitch to draw the bone on either side of the bone hinge together to aid compression. In particular, the thread pitch may be narrower towards the first end of the screw (the proximal end) and wider towards the second end (the distal end). The screw may be headless screw such as a headless compression screw, e.g. "Fixos 2" sold by Stryker Corporation (United States Patent No. 9,011,505 B2) or a Bio-Compression screw as sold Arthrex.

The screw can be a solid non-cannulated screw or a cannulated screw. More preferably the implant is a cannulated screw. A cannulated screw can be guided over the bone hinge using a guide wire, which is discussed further below. The screw may have at least two threaded parts, one at the first end and one at the second end or may have a thread extending from the first end to the second end. The screw may be tapered.

The screw can be made of plastic, metal or biodegradable material.

In an alternative example, the implant can be a pulley thread, anchored by two plates on the surface of the bone, one at the first end and one at the second end of the implant.

The size of the implant is not particularly limited but will be chosen based on the size of the bone involved in the surgery.

The method may comprise inserting a guide to establish the desired position for the implant. The implant may then be inserted along the position established by the guide. In a preferred example the guide is a wire (e.g. a K wire / Kirschner wire). The position established by the guide may be over drilled to allow the insertion of the implant. If the implant is cannulated this can be inserted over the K wire.

The guide may be inserted before the bone is cut in order to mark the position of the area of bone that is to remain uncut and be the bone hinge. Alternatively, the guide may be inserted after the bone is cut either before or after the aperture is created and fixed. Preferably the guide is inserted after the bone is cut but before the aperture is created.

The method comprises applying a compressive force between the first end and the second end of the implant across the bone hinge. In particular, this can be accomplished by tightening the implant, e.g. by tightening the screw or by tightening the pulley thread.

In a second aspect, the present disclosure provides a similar method to the one described above as the first aspect, with the exception that compression across the bone hinge is achieved by inserting an implant across the cut in the bone at a position adjacent to the bone hinge.

In particular, the method for compressing a bone hinge created during surgery to re-align a bone in a subject according to the second aspect of the disclosure comprises:
making a cut in the bone from a second point on the outer surface of the bone to create a first segment of bone and a second segment of bone that are separated by the cut, wherein the cut is a partial cut that terminates at the bone hinge such that the first and second segments of bone remain joined at the bone hinge,
inserting an implant comprising a first end and an opposing second end into the bone from a first point on an outer surface of the bone, wherein the first end remains at the first point and the second end is inserted across the cut adjacent to the bone hinge to the opposite side of the cut; and
applying a compressive force between the first end and the second end across the cut, so as to compress the bone hinge.

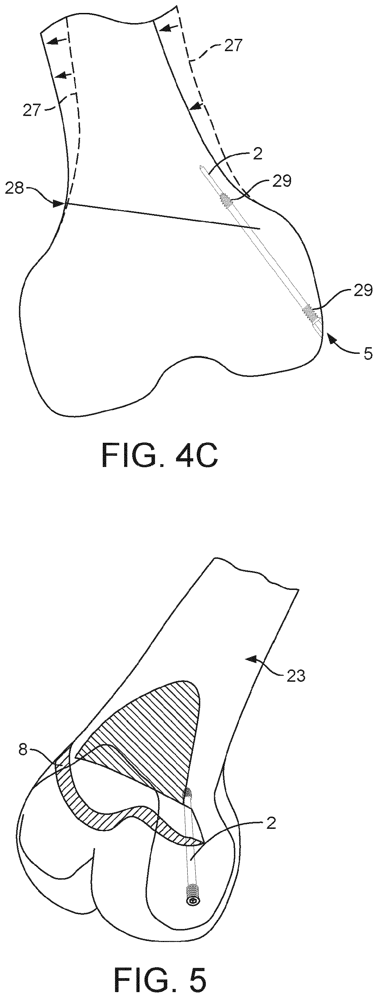

In particular, as shown in Figure 4C and in Figure 5, the implant is inserted across the cut at a position that is adjacent to the bone hinge, and is then used to indirectly apply a compressive force across the bone hinge. A position closer to the bone hinge will achieved better compression. Therefore, the implant may traverse the cut at a position that is within the final 20% of the length of the cut, preferably within the final 10% of the length of the cut.

The implant may be inserted obliquely across the cut or at an angle of 90°. As with the method of the first aspect of the invention, the positioning of the implant can be selected in order to achieve as much compression of the bone hinge as possible given the anatomical constraints set by the specific osteotomy being performed.

The implant is inserted across the cut and into the bone on the opposite side. In particular, the implant should extend at least 1-2 mm, preferably at least 5 mm beyond the cut or beyond the wedge where the osteotomy is an opening wedge osteotomy. How far the second end of the implant extends beyond this is not particularly limited as described above for the first aspect of the disclosure.

Where the osteotomy is an opening wedge osteotomy (as shown in Figure 5) the implant can be inserted across the aperture (or across the bone graft or artificial material in the aperture where this is used) and into the bone on the opposite side.

The features described above for the method according to the first aspect of the disclosure apply equally, where compatible, to the method according to the second aspect of the disclosure.

The present invention provides a device that is suitable for use during osteotomy surgery and which can be used in a method according to the first aspect of the disclosure described above. In particular, the device can be used once the bone cut from the second point is created, to insert the guide or implant across the bone hinge. The features described above in relation to the method of the first aspect of the disclosure also apply to the description of the device provided below.

In particular, the present invention provides a device suitable for use during surgery to direct the insertion of a guide (10) or an implant (2) across a bone hinge (1), wherein the bone hinge is adjacent to a cut (6) made in a bone during the surgery, the device comprising:
(i) a main body (19) comprising or consisting of a first arm (11), wherein the first arm comprises a first opening (12) for receiving the guide or the implant; and
(iii) an insertion part (13) having a first end and a second end, the first end comprising an elongated portion (14) which is suitable for insertion into the cut, and the second end comprising an attachment part for reversibly attaching the insertion part to the main body;
wherein when the elongated portion is inserted into the cut and the insertion part is attached to the main body, the first opening is configured to direct the insertion of the guide or the implant into the bone from a first point on the outer surface across the bone hinge.

In one example the main body comprises a second arm (15), the second arm extending away from the first arm, wherein the second arm comprises a part, preferably a second opening (16), for receiving a joining part (17) to join the device to the bone during the surgery at a position on the opposite side of the cut to the first point.

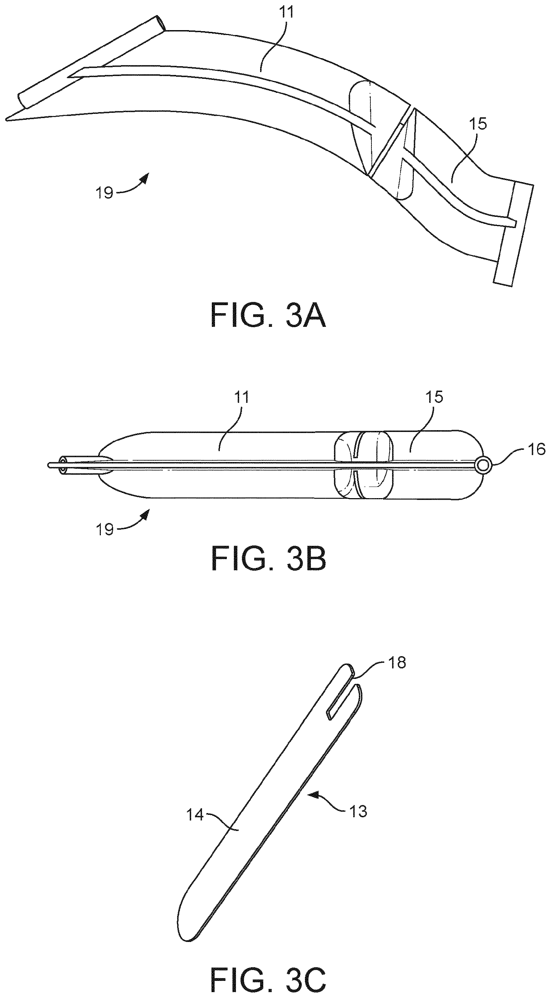

In particular, the device is assembled during the surgery from a plurality of parts. The plurality of parts include a main body comprising or consisting of a first arm, an optional second arm, and an insertion part, where a portion of the insertion part is to be inserted into the bone cut. An example of a main body (19) of the device comprising a first arm (11) and a second arm (15) is shown in Figures 3A and 3B.

The insertion part has a first end and a second end. The first end comprises an elongated portion which is of a suitable size (i.e. width, depth and length) for insertion into the bone cut. Preferably the elongate portion is an elongate flat portion. The second end comprises an attachment part for reversibly attaching the insertion part to the main body.

In one example the elongated portion has a tip comprising a metal or metallic portion. As such, the tip can be seen on any modality of imaging, such as X-ray or fluoroscope images of the bone during the surgery. Where the elongate portion is inserted to the full depth of the bone cut, the tip will be adjacent to the end of the cut and the area of the bone hinge. This can be used to assist in insertion of the guide.

The attachments part is not particularly limited but is required to provide a secure but temporary attachment to the main body, allowing the insertion part to be easily detached from the main body once the guide or the implant has been inserted into the bone. In one example the attachment part of the insertion part is a slit (18) which fits into the main body in order to be reversibly attached thereto. An example of such an insertion part is shown in Figure 3C.

At one end, distal from the part of the main body that attaches to the insertion part, the second arm comprises a part, preferably a second opening, for receiving a joining part for joining or securing the device to the bone during the surgery. In particular, the second opening can be a hollow tube or channel into which the securing part can be inserted. (An example is shown as part (16) in Figure 3B.) Preferably the securing part is a wire or pin (e.g. a K wire). Where the part is a second opening it is preferred that this is sized to fit a K wire.

At one end, distal from the part of the main body that attaches to the insertion part, the first arm comprises a first opening for receiving the guide or implant during the surgery. In particular, the opening can be a hollow tube or channel into which the guide or the implant can be inserted and directed into the bone. Preferably the guide is a wire or pin (e.g. a K wire). Preferably the implant is a screw, most preferably a cannulated compression screw. Most preferably the first opening is sized to fit a K wire.

The first opening is arranged to direct the guide or implant into the bone on a plane which intersects with the bone hinge, e.g. which intersects with the theoretical line (or plane) that represents the shortest distance between the point at which the cut terminates and the cortex, or which intersects with the direction of cutting at termination of the bone cut, as described above.

In particular, when the device is arranged so that the elongate portion is inserted into the cut and the insertion part is attached (via the attachment part) to the main body (and optionally the joining part is also attached to the second arm and secured to the bone), the configuration of the first opening directs the guide or implant (positioned in the first opening) into the bone on the plane which intersects with the theoretical line referred to above. This allows the device to be used to position the guide or the implant correctly. Normally, the configuration of the first opening directs the guide or implant at an angle of 20° to 50° to the elongated portion when the insertion part is attached to the main body, more preferably at an angle between 25° and 35°. For a device which is to be used in a high tibial osteotomy in which the implant is to be inserted from the same side of the bone as the cut, an angle of between 28° and 32° or approximately 30° can be used. However, any angle that achieves compression of the bone hinge is acceptable.

For a device in which the main body comprises a second arm, the part, preferably a second opening, for receiving the joining part may be configured to direct the joining part to attach to the bone at an angle of around 15° to 30° to the elongated portion when the insertion part is attached to the main body, preferably at an angle of 18° to 22° or approximately 20°.

As indicated above, the device of the present invention can be used in the method of surgery to assist in positioning of the guide and/or insertion of the implant. In particular, during the method of surgery once the partial cut is created the elongated flat portion of the insertion part can be inserted into the cut and the insertion part attached to the main body of the device using the attachment part at the second end. Where the device comprises a second arm, a joining part (such as a K wire) can be used to join or secure the device to the bone at a point removed from the cut. Once the insertion part is inserted into the bone cut, and optionally the joining part is joined or secured to the bone above the cut, a stable base is provided from which the guide or the implant can be inserted. The guide or implant can then be placed in the first opening of the first arm, inserted into the bone at the first point (usually by drilling, or by tapping) and positioned across the bone hinge. Where the elongated portion of the insertion part comprises a metallic or metal tip, this insertion can be done under fluoroscopic control in order to achieve the correct placement of the tip.

Once the guide or implant are at least partially in place the insertion part can be detached from the main body, and the optional joining part detached from the bone. The device can then be removed from the guide or implant. Further insertion of the guide or implant can be performed if necessary. If a guide has been inserted this then works as a temporary device to be over drilled to bring the implant in, which is then is placed into the bone.

Once the implant is in place it can be used to apply compression across the bone hinge as described above.

In a further aspect the present invention provides a sterile surgical package comprising the main body and the insertion part of the device described above. The package may comprise one or more of: (i) an implant also as described above; (ii) at least one guide as defined above; and (iii) a joining part as described above. Preferably the package comprises at least one guide and/or at least one joining part More preferably the package comprises at least two K wires and the device comprises a first opening and a second opening that are sized to fit the K wires - one as a joining part and one as a guide. The package may be a single-use package that is provided in a sterile condition so that it can be directly used in surgery.

The following are intended as examples only and do not limit the present disclosure.

### Examples

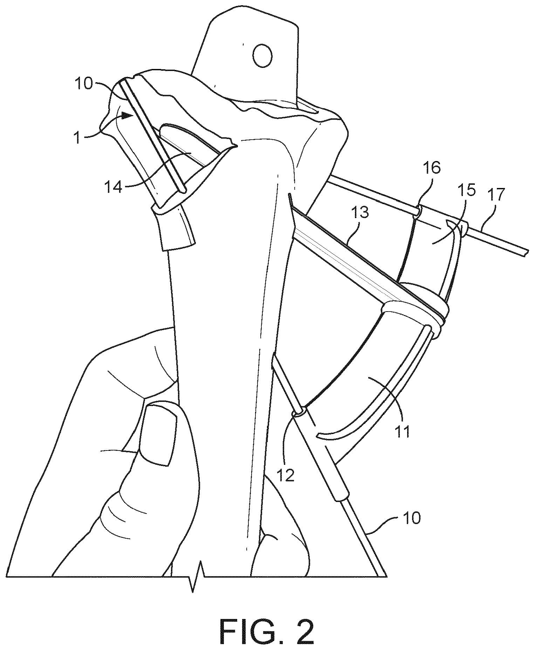

An example of the device of the present disclosure has been made and tested using a plastic model of a tibia as shown in Figure 2. In particular, suitable plastic bone models that are routinely used for high tibial osteotomy training can be obtained from Synbone AG (Switzerland).

As shown in Figure 2 a device comprising a main body with a first arm (11), second arm (15) and insertion part (13) which includes an elongated portion (14) was made using a 3D printer. A partial cut suitable for performing a high tibial osteotomy was made into the bone, leaving an uncut region of bone hinge (1). The elongate portion (14) of the insertion part (13) was inserted into the cut and the insertion part attached to the main body of the device, between the first and second arms.

The second arm (15) comprised an opening (16) into which a K wire joining part (17) was inserted which was then inserted into the bone in order to secure the device to the bone. The first opening (12) on the first arm (11) was then utilised to position the guide (10) K-wire across the bone hinge. In this example, the device directs the guide K-wire into the bone at an angle that is at 30° to the insertion part inserted into the cut, and the K-wire as the joining part is directed into the bone by the second arm at an angle of 20° to the insertion part inserted into the cut.

Other variants or use cases of the disclosed techniques may become apparent to the person skilled in the art once given the disclosure herein. The disclosure is not limited by the described embodiments but only by the accompanying claims.

## Claims

1. A device suitable for use during surgery to direct the insertion of a guide (10) or an implant (2) across a bone hinge (1), wherein the bone hinge (1) is adjacent to a cut (7) made in a bone during the surgery, the device comprising:
(i) a main body (19) comprising or consisting of a first arm (11), wherein the first arm (11) comprises a first opening (12) for receiving the guide (10) or the implant (2); and
(iii) an insertion part (13) having a first end and a second end, the first end comprising an elongated portion (14) which is suitable for insertion into the cut (7), and the second end comprising an attachment part for reversibly attaching the insertion part to the main body (19);
wherein when the elongated portion (14) is inserted into the cut and the insertion part (13) is attached to the main body (19), the first opening (12) is configured to direct the insertion of the guide (10) or the implant (2) into the bone from a first point (6) on the outer surface across the bone hinge (1).

2. The device according to claim 1, wherein the main body (19) comprises a second arm (15), the second arm (15) extending away from the first arm (11), wherein the second arm (15) comprises a part (16), preferably a second opening, for receiving a joining part (17) to join the device to the bone during the surgery at a position on the opposite side of the cut to the first point.

3. The device according to claim 1 or claim 2, wherein a tip of the elongated portion (14) comprises a metal or metallic section.

4. The device according to any of claims 1 to 3, wherein the elongated portion (14) is suitable for insertion to the end of the cut (7), such that a tip of the elongated portion (14) is adjacent to the bone hinge (1).

5. The device according to any preceding claim , wherein the first opening (11) and/or the second opening (16) are independently selected from a hollow tube or a channel.

6. The device according to any preceding claim, wherein the first opening is adapted to receive a K-wire as the guide and/or wherein the second opening (16) is adapted to receive a K-wire as the joining part.

7. The device according to any preceding claim, wherein the attachment part of the second end of the insertion part is a slit which fits into the main body so as to reversibly attach the insertion part to the main body.

8. The device according to any preceding claim, wherein the first opening comprises a hollow tube or channel which is at an angle of 20° to 50° to the elongated portion when the insertion part is attached to the main body.

9. A sterile surgical package comprising the main body and the insertion part of the device according to any one of claims 1 to 7, optionally wherein the package comprises one or more of: (i) an implant; and (ii) at least one guide;
wherein the implant is suitable for applying compression across the bone hinge, and wherein the guide is a wire or pin.

## Patentansprüche

1. Vorrichtung, die zur Verwendung während eines chirurgischen Eingriffs geeignet ist, um das Einführen einer Führung (10) oder eines Implantats (2) über ein Knochenscharnier (1) zu lenken, wobei das Knochenscharnier (1) an einen Schnitt (7) angrenzt, der in einem Knochen während des chirurgischen Eingriffs vorgenommen wird, wobei die Vorrichtung umfasst:
(i) einen Hauptkörper (19), umfassend oder bestehend aus einem ersten Arm (11), wobei der erste Arm (11) eine erste Öffnung (12) zum Aufnehmen der Führung (10) oder des Implantats (2) umfasst; und
(iii) einen Einführungsteil (13) mit einem ersten Ende und einem zweiten Ende, wobei das erste Ende einen länglichen Abschnitt (14) umfasst, der zum Einführen in den Schnitt (7) geeignet ist, und das zweite Ende einen Anbringungsteil zum reversiblen Anbringen des Einführungsteils an dem Hauptkörper (19) umfasst;
wobei, wenn der längliche Abschnitt (14) in den Schnitt eingeführt ist und der Einführungsteil (13) an dem Hauptkörper (19) angebracht ist, die erste Öffnung (12) dazu konfiguriert ist, das Einführen der Führung (10) oder des Implantats (2) in den Knochen von einem ersten Punkt (6) auf der Außenfläche über das Knochenscharnier (1) zu lenken.

2. Vorrichtung nach Anspruch 1, wobei der Hauptkörper (19) einen zweiten Arm (15) umfasst, wobei der zweite Arm (15) sich von dem ersten Arm (11) weg erstreckt, wobei der zweite Arm (15) einen Teil (16), vorzugsweise eine zweite Öffnung, zum Aufnehmen eines Verbindungsteils (17) umfasst, um die Vorrichtung während eines chirurgischen Eingriffs mit dem Knochen an einer Position auf der entgegengesetzten Seite des Schnitts zu dem ersten Punkt zu verbinden.

3. Vorrichtung nach Anspruch 1 oder Anspruch 2, wobei eine Spitze des länglichen Abschnitts (14) eine Metall- oder metallischen Sektion umfasst.

4. Vorrichtung nach einem der Ansprüche 1 bis 3, wobei der längliche Abschnitt (14) zum Einführen an dem Ende des Schnitts (7) geeignet ist, so dass eine Spitze des länglichen Abschnitts (14) an das Knochenscharnier (1) angrenzt.

5. Vorrichtung nach einem vorhergehenden Anspruch, wobei die erste Öffnung (11) und/oder die zweite Öffnung (16) unabhängig aus einer hohlen Röhre oder einem Kanal ausgewählt sind.

6. Vorrichtung nach einem vorhergehenden Anspruch, wobei die erste Öffnung dazu eingerichtet ist, einen K-Draht als die Führung aufzunehmen, und/oder wobei die zweite Öffnung (16) dazu eingerichtet ist, einen K-Draht als den Verbindungsteil aufzunehmen.

7. Vorrichtung nach einem vorhergehenden Anspruch, wobei der Anbringungsteil des zweiten Endes des Einführungsteils ein Schlitz ist, der in den Hauptkörper passt, um den Einführungsteil reversibel an dem Hauptkörper anzubringen.

8. Vorrichtung nach einem vorhergehenden Anspruch, wobei die erste Öffnung eine hohle Röhre oder einen Kanal umfasst, die bzw. der in einem Winkel von 20° bis 50° zu dem länglichen Abschnitt ist, wenn der Einführungsteil an dem Hauptkörper angebracht ist.

9. Sterile chirurgische Packung, umfassend den Hauptkörper und den Einführungsteil der Vorrichtung nach einem der Ansprüche 1 bis 7, optional wobei die Packung eines bzw. eine oder mehrere von: (i) einem Implantat und (ii) mindestens einer Führung umfasst;
wobei das Implantat zum Anwenden einer Kompression über das Knochenscharnier geeignet ist
und wobei die Führung ein Draht oder ein Stift ist.

## Revendications

1. Un dispositif approprié pour être utilisé pendant une intervention chirurgicale pour diriger l'insertion d'un guide (10) ou d'un implant (2) dans une articulation d'os (1), dans lequel l'articulation de l'os (1) est adjacente à une incision (7) faite dans un os pendant l'intervention chirurgicale, le dispositif comprenant :
(i) un corps principal (19) comprenant ou constitué d'un premier bras (11), dans lequel le premier bras (11) comprend une première ouverture (12) pour recevoir le guide (10) ou l'implant (2) ; et
(ii) une partie d'insertion (13) ayant une première extrémité et une deuxième extrémité, la première extrémité comprenant une partie allongée (14) qui est appropriée pour une insertion dans l'incision (7), et la deuxième extrémité comprenant une partie de fixation pour fixer de manière réversible la partie d'insertion au corps principal (19) ;
dans lequel quand la partie allongée (14) est introduite dans l'incision et que la partie d'insertion (13) est fixée au corps principal (19), la première ouverture (12) est configurée pour diriger l'insertion du guide (10) ou de l'implant (2) dans l'os depuis un premier point (6) sur la surface extérieure de l'articulation de l'os (1).

2. Le dispositif selon la revendication 1, dans lequel le corps principal (19) comprend un deuxième bras (15), le deuxième bras (15) s'étendant en s'écartant du premier bras (11), dans lequel le deuxième bras (15) comprend une partie (16), de préférence une deuxième ouverture, pour recevoir une partie de raccordement (17) pour raccorder le dispositif à l'os pendant l'intervention chirurgicale à une position sur le côté opposé de l'incision au premier point.

3. Le dispositif selon la revendication 1 ou la revendication 2, dans lequel une pointe de la partie allongée (14) comprend un métal ou une section métallique.

4. Le dispositif selon l'une quelconque des revendications 1 à 3, dans lequel la partie allongée (14) est appropriée pour une insertion à l'extrémité de l'incision (7), de telle sorte qu'une pointe de la partie allongée (14) est adjacente à l'articulation de l'os (1).

5. Le dispositif selon l'une quelconque des revendications précédentes, dans lequel la première ouverture (11) et/ou la deuxième ouverture (16) sont sélectionnées indépendamment parmi un tube creux ou un canal.

6. Le dispositif selon l'une quelconque des revendications précédentes, dans lequel la première ouverture est adaptée pour recevoir une broche de Kirschner en tant que guide et/ou dans lequel la deuxième ouverture (16) est adaptée pour recevoir une broche de Kirschner en tant que partie de raccordement.

7. Le dispositif selon l'une quelconque des revendications précédentes, dans lequel la partie de fixation de la deuxième extrémité de la partie d'insertion est une fente qui s'ajuste dans le corps principal de façon à fixer de manière réversible la partie d'insertion au corps principal.

8. Le dispositif selon l'une quelconque des revendications précédentes, dans lequel la première ouverture comprend un tube creux ou un canal qui est à un angle de 20° à 50° de la partie allongée quand la partie d'insertion est fixée au corps principal.

9. Un kit chirurgical stérile comprenant le corps principal et la partie d'insertion du dispositif selon l'une quelconque des revendications 1 à 7, en option dans lequel le kit comprend un ou plusieurs éléments parmi : (i) un implant ; et (ii) au moins un guide ;
dans lequel l'implant est approprié pour exercer une compression sur l'articulation de l'os, et
dans lequel le guide est un fil métallique ou une goupille.
